(19) **Europäisches Patentamt European Patent Office Office européen des brevets**

(11) **EP 4 656 154 A1**

(12) **EUROPEAN PATENT APPLICATION**
published in accordance with Art. 153(4) EPC

(43) Date of publication:
   **03.12.2025 Bulletin 2025/49**

(51) International Patent Classification (IPC):
   ***A61B 34/20*** (2016.01)

(21) Application number: **23918684.4**

(52) Cooperative Patent Classification (CPC):
   **A61B 34/20**

(22) Date of filing: **27.12.2023**

(86) International application number:
   **PCT/JP2023/047014**

(87) International publication number:
   **WO 2024/157730 (02.08.2024 Gazette 2024/31)**

(84) Designated Contracting States:
   **AL AT BE BG CH CY CZ DE DK EE ES FI FR GB
   GR HR HU IE IS IT LI LT LU LV MC ME MK MT NL
   NO PL PT RO RS SE SI SK SM TR**
   Designated Extension States:
   **BA**
   Designated Validation States:
   **KH MA MD TN**

(30) Priority: **27.01.2023 PCT/JP2023/002678**

(71) Applicant: **Tohoku University
   Sendai-shi, Miyagi 980-8577 (JP)**

(72) Inventors:
   • **MIYAGUCHI Hiroshi
     Sendai-shi, Miyagi 980-8577 (JP)**
   • **HARIGAI Ayaka
     Sendai-shi, Miyagi 980-8577 (JP)**
   • **OGURO Sota
     Sendai-shi, Miyagi 980-8577 (JP)**
   • **ITO Takahiro
     Sendai-shi, Miyagi 980-8577 (JP)**

(74) Representative: **Cabinet Becker et Associés
   25, rue Louis le Grand
   75002 Paris (FR)**

(54) **PUNCTURE SYSTEM, PUNCTURE ASSISTING TOOL, BODY SURFACE-IRRADIATING LASER MECHANISM, AND PUNCTURE NAVIGATION SYSTEM**

(57) A puncture system (1) includes: a puncture assisting tool (3) including a holding portion attached to or detached from a puncture needle (2) that punctures using a line (H) formed at a body surface of a patient (M) by a vertical plane including an insertion point (P) and a target point (Q) in a body of the patient (M), and a sensor unit (32) that measures an angle of the held puncture needle from a vertical axis or a horizontal plane; a body surface-irradiating laser mechanism (4) including a laser irradiation unit (42) that emits laser to the vertical plane along the line (H) formed at the body surface of the patient, and a movement mechanism that moves the laser irradiation unit; and a puncture navigation system including a calculation unit that determines an irradiation position of the laser and an insertion angle of the puncture needle (2) based on the insertion point and the target point (Q) in the body of the patient (M), and a control unit that controls the laser irradiation unit.

FIG. 1

EP 4 656 154 A1

## Description

Technical Field

The present invention relates to a puncture assisting tool, a body surface-irradiating laser mechanism, a puncture navigation system, and a puncture system applied to biopsy, drainage, radio frequency ablation, cryotherapy, and the like, where a body is punctured with a needle using a CT-guided puncture, an ultrasound-guided puncture, an MRI-guided puncture, or the like.

The present application claims priority to PCT/JP2023/002678 filed on January 27, 2023, the contents of which are hereby incorporated by reference.

Background Art

In related art, in a case where a puncture needle is penetrated (inserted) toward a puncture target point in a patient specified in a computed tomography (CT) cross section using CT to perform a puncture, the puncture needle is located in the CT cross section such that laser is emitted to a back side in an advancing direction of the puncture needle (for example, see PTLs 1 and 2).

PTL 1 also discloses that an insertion depth, a transverse insertion angle, and a craniocaudal insertion angle of the needle to be inserted into a body from an insertion entry point to a target in the body are determined, and laser is emitted toward the insertion entry point.

An angle of a puncture needle when a practitioner inserts the puncture needle into a patient is also presented (for example, see PTLs 3 to 5).

It is also known to detect a target organ to be punctured with an ultrasound probe, and to insert a puncture needle using line laser from the ultrasound probe as a guide (for example, see PTL 6).

It is also known to perform a puncture, using a housing to which line-generating laser and a puncture needle are integrally attached, by aligning a laser beam generated by a CT scanner with a visible laser beam of the line-generating laser attached to the housing (for example, see PTL 7).

Further, it is known to use intersecting beams from two laser planes in order to mark a guide path of a medical instrument such as a puncture needle or a catheter (for example, see PTL 8).

Citation List

Patent Literature

PTL 1: JP2002-511784A
PTL 2: JP2000-070272A
PTL 3: JP2009-523508A
PTL 4: CN1939234B
PTL 5: WO2017/070124
PTL 6: US2010/0030082
PTL 7: US2016/0296179
PTL 8: US5,782,842B

Summary of Invention

Technical Problem

An apparatus that emits the laser to the back side in the advancing direction of the puncture needle as disclosed in PTLs 1 and 2 is highly susceptible to positional deviation since the laser is emitted to one point from the entry point toward the target point. In particular, when the puncture is performed with the puncture needle, the patient inevitably moves, and at that time, it is difficult to re-correct the laser.

In addition, in order to emit the laser to the back side in the advancing direction of the puncture needle, a special puncture needle whose back side is formed in this way is required. There have been problems in that an ablation needle or a puncture needle used in cryotherapy, in which a cable, a fluid delivery tube, or the like is provided at a rear end of the puncture needle, cannot be used.

In addition, since it is necessary to emit the laser from above, it is difficult to emit the laser depending on an angle, and a puncture range may be limited or a size of the apparatus may be increased.

In PTL 1, the insertion depth, the transverse insertion angle, and the craniocaudal insertion angle of the needle are determined, and the laser is emitted toward the insertion entry point, but this requires the patient to be located at an accurate position relative to the laser, and correction cannot be made when the patient moves, which causes difficulties.

An instrument for presenting the angle of the puncture needle as disclosed in PTLs 3 to 5 does not perform laser guidance, and instead repeats imaging using CT or the like to perform a puncture at a predetermined angle, and therefore, at the time of CT imaging, the practitioner needs to keep holding an instrument for holding the puncture needle, and thus significant X-ray exposure is unavoidable, and since the imaging is repeated several times using CT or the like, not only the practitioner but also the patient is unavoidably exposed to a large amount of X-ray radiation.

In addition, although an accurate puncture angle of the puncture needle is presented, there may be a case where the puncture needle is moved by a slice thickness of a CT image in a shape along a CT slice plane while maintaining an insertion angle to perform a puncture in a medical procedure, and the medical procedure corresponding to such a case cannot be performed.

In PTL 6, the puncture needle is inserted using the line laser as a guide and the line laser is emitted from the ultrasound probe, and therefore, hand shaking or the like cannot be avoided and it is difficult to accurately maintain the line laser guidance, and thus an accurate puncture is fairly difficult. In addition, it is fairly difficult for a practitioner who inserts the puncture needle to hold the ultrasound probe and puncture in a state where accurate line laser guidance is displayed, and thus an assistant is required. Since the line laser is emitted from the ultrasound probe, a degree of freedom of the laser is extremely low, and it is difficult to perform appropriate laser guidance.

Further, the line laser from the ultrasound probe pressed against a body surface of the patient is easily affected by unintended body movement of the patient, a line laser position easily deviates, and it is extremely difficult to perform a safe puncture.

In addition, in PTL 6, the laser beam generated by the CT scanner and the visible laser beam of the line-generating laser attached to the housing are aligned to perform the puncture, but since the practitioner needs to operate while holding the housing with a hand, it is difficult to hold the visible laser beam at the same position as the laser generated by the CT scanner. In addition, since the line-generating laser and the puncture needle are integrated, a burden on the practitioner is large and usability is poor.

In PTL 7, the puncture needle is guided along a puncture path formed by the intersecting beams from the two laser planes, and since it is necessary to accurately form the two intersecting laser planes at a puncture position of a patient, there is a problem that an apparatus is likely to be large and a practitioner needs to perform a medical procedure without blocking the two laser beams. There is also a problem of being highly susceptible to movement of the patient.

The invention has been made in view of the above problems, and an object thereof is to provide a puncture system, a puncture assisting tool, a body surface-irradiating laser mechanism, and a puncture navigation system that can easily and accurately check a tilt angle of a puncture needle with which a patient is punctured with a small burden on a practitioner, and can perform a puncture with high accuracy.

Solution to Problem

A puncture system according to the invention includes: a puncture assisting tool including a holding portion attached to or detached from a puncture needle that punctures using a line formed at a body surface of a patient by a vertical plane including an insertion point and a target point in a body of the patient, and a sensor unit that measures an angle of the held puncture needle from a vertical axis or a horizontal plane; a body surface-irradiating laser mechanism including a laser irradiation unit that emits laser to the vertical plane along the line formed at the body surface of the patient, and a movement mechanism that moves the laser irradiation unit; and a puncture navigation system including a calculation unit that determines an irradiation position of the laser and an insertion angle of the puncture needle based on the insertion point and the target point in the body of the patient, and a control unit that controls the laser irradiation unit.

A puncture assisting tool according to the invention is a puncture assisting tool attachable to and detachable from a puncture needle that punctures using a line formed at a body surface of a patient by a vertical plane including an insertion point and a target point in a body of the patient, the puncture assisting tool including: a holding portion including a fixed portion that holds the puncture needle, a movable portion, and a fixing mechanism that holds and fixes the puncture needle with the fixed portion and the movable portion; a sensor unit configured to measure an angle of the held puncture needle from a vertical axis or a horizontal plane; and a presentation unit configured to present the angle, in which the fixed portion has a perpendicular surface with respect to the puncture needle when the puncture needle is fixed, and a scale is marked on the perpendicular surface.

A body surface-irradiating laser mechanism according to the invention includes: a holding member; a laser irradiation unit attached via the holding member and configured to emit laser along a line formed at a body surface of a patient by a vertical plane including an insertion point and a target point in a body of the patient toward the vertical plane; and a movement mechanism configured to move the laser irradiation unit provided at the holding member.

A puncture navigation system according to the invention includes: a calculation unit configured to determine an insertion angle and an insertion length based on an insertion point and a target point in a body of a patient specified using at least one of CT, MRI, and an ultrasound probe, and to calculate an angle from a body axis of the patient relative to a plane including the target point and the insertion point; and a control unit configured to control, based on the angle from the body axis of the patient calculated by the calculation unit, a laser irradiation unit that emits laser to a vertical plane along a line formed at a body surface of the patient by the vertical plane.

Advantageous Effects of Invention

According to the puncture system, the puncture assisting tool, the body surface-irradiating laser mechanism, and the puncture navigation system according to each aspect of the invention, a tilt angle of a puncture needle with which a patient is punctured can be easily and accurately checked with a small burden on a practitioner, and a puncture can be performed with high accuracy.

Brief Description of Drawings

[FIG. 1] FIG. 1 is a perspective view showing an overall overview of a puncture system according to a first embodiment.
[FIG. 2] FIG. 2 is a perspective view showing a puncture assisting tool holding a puncture needle.
[FIG. 3] FIG. 3 is a perspective view showing a specific configuration of the puncture assisting tool.
[FIG. 4] FIG. 4 is a perspective view showing a positional relationship between a puncture needle and the puncture assisting tool during a puncture.
[FIG. 5] FIG. 5 is a side view of FIG. 4.
[FIG. 6] FIG. 6 is a perspective view showing an overall configuration of a body surface-irradiating laser mechanism.
[FIG. 7] FIG. 7 is a perspective view showing a configuration of a movement mechanism of the body surface-irradiating laser mechanism shown in FIG. 6.
[FIG. 8] FIG. 8 is a plan view showing a calibration mechanism for matching coordinates of a puncture navigation system with coordinates of a CT apparatus.
[FIG. 9] FIG. 9 is a plan view showing the puncture navigation system.
[FIG. 10] FIG. 10 is a perspective view showing a specific configuration of a puncture assisting tool according to a third embodiment.
[FIG. 11] FIG. 11 is a perspective view showing a positional relationship between a puncture needle and the puncture assisting tool during a puncture.
[FIG. 12] FIG. 12 shows a vertical plane including an Nx axis in FIG. 11.
[FIG. 13] FIG. 13 shows an Ny-axis-Nz-axis plane in FIG. 11.
[FIG. 14] FIG. 14 shows an overview of a puncture system according to a fifth embodiment using an ultrasound probe in the puncture system.
[FIG. 15] FIG. 15 shows a configuration of the puncture system according to the fifth embodiment.
[FIG. 16] FIG. 16 shows the configuration of the puncture system according to the fifth embodiment.
[FIG. 17] FIG. 17 shows a configuration of an adjustment mechanism of a body surface-irradiating laser mechanism according to a sixth embodiment.

Description of Embodiments

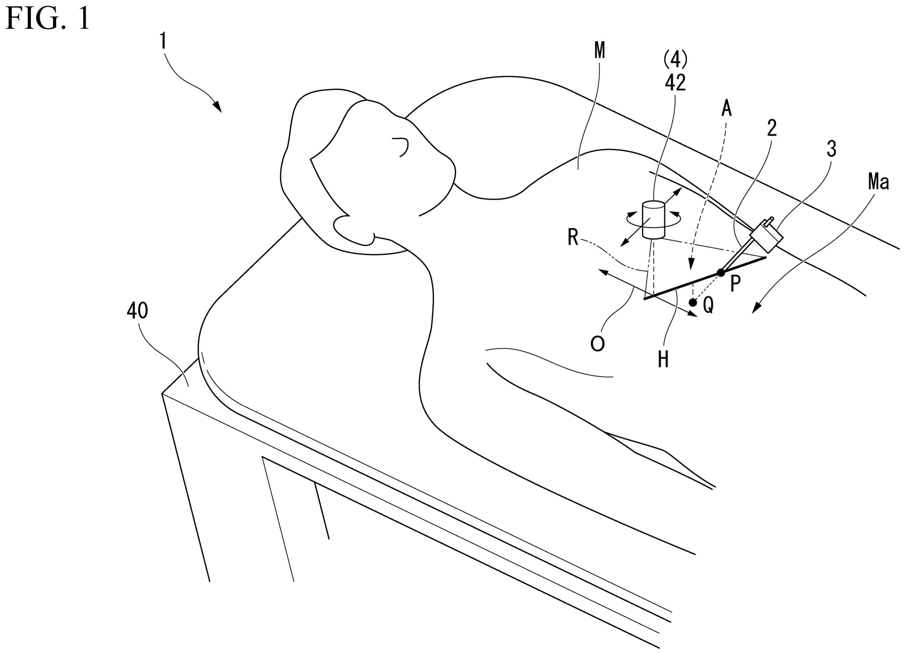

Examples of a puncture system, a puncture assisting tool, a body surface-irradiating laser mechanism, and a puncture navigation system according to embodiments of the invention will be described with reference to FIGS. 1 to 9.
In the present description, there are expressions such as puncturing along a line, aligning positions, and the like, but such expressions are not necessarily limited to complete matching, and it is understood that the expressions include a range of errors allowed in a general medical procedure.

[First Embodiment]

[Puncture System]

FIG. 1 is a perspective view showing a puncture system 1 in the embodiment, and shows a state inside a gantry of a computed tomography (CT) apparatus (not shown). As shown in FIG. 1, the puncture system 1 is used for puncturing a body of a patient M with a puncture needle 2 in a CT-guided puncture, an ultrasound-guided puncture, an MRI-guided puncture, or the like. In the first embodiment, an example in which the CT-guided puncture is used will be described. That is, the puncture system 1 is used to display, using a body surface-irradiating laser mechanism 4, a body surface marker line H at a body surface Ma of the patient M in a plane including a puncture insertion point P and a puncture target point Q in the body of the patient M determined in advance by CT, and to puncture with the puncture needle 2 along the body surface marker line H with high accuracy. The body surface Ma may be a chest, a ventral side, or a dorsal side. O in FIG. 1 represents a body axis.

In the invention, the body axis O means a normal body axis of the patient M analyzed based on the patient M obtained by imaging (a long axis around which the body is approximately symmetrical and which extends from a caudal end to a cranial end), the patient M is generally placed at a center of a medical table 40, and since movement of the patient M can be eliminated, a central axis in a longitudinal direction of the medical table 40 may be the body axis O of the patient. The body axis O is not limited to the above, and may be a reference fixed axis (reference axis) determined as a reference when the patient M or the like is subjected to a medical procedure.

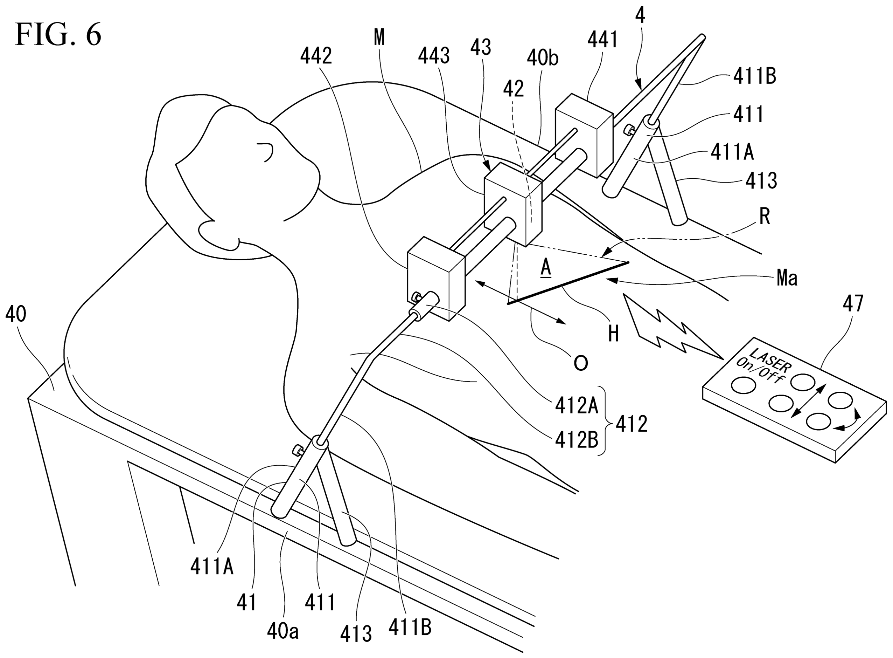

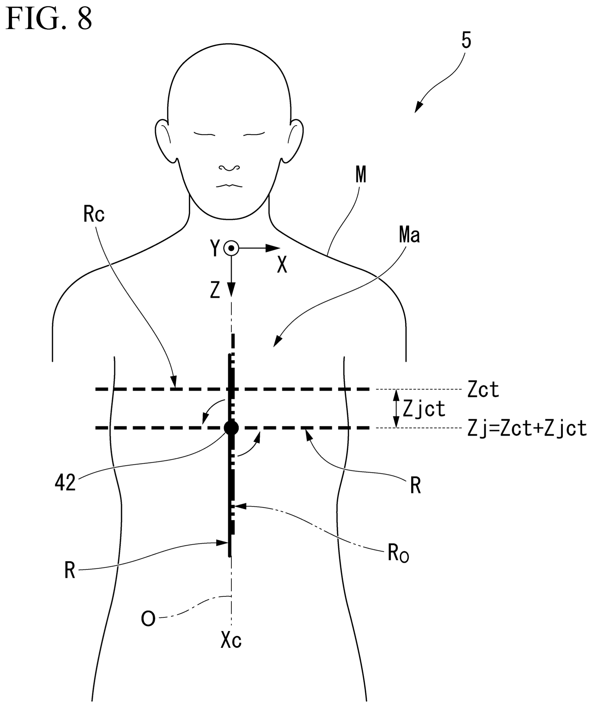

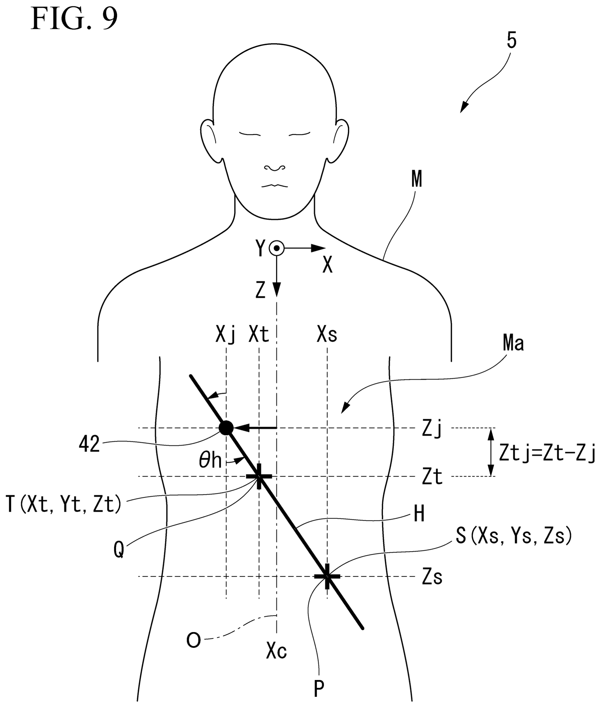

The puncture system 1 includes a puncture assisting tool 3 detachably attached to the puncture needle 2, the body surface-irradiating laser mechanism 4 (see FIG. 6) that emits laser light R to the body surface Ma of the patient M, and a puncture navigation system 5 that determines a position of the laser light R, which is not shown in FIG. 1 (see FIGS. 8 and 9).

As described above, the puncture system 1 uses the puncture target point Q in the body of the patient M, which is determined by CT. A method for determining the puncture target point Q using CT will be described later in the puncture navigation system 5.

[Puncture Assisting Tool]

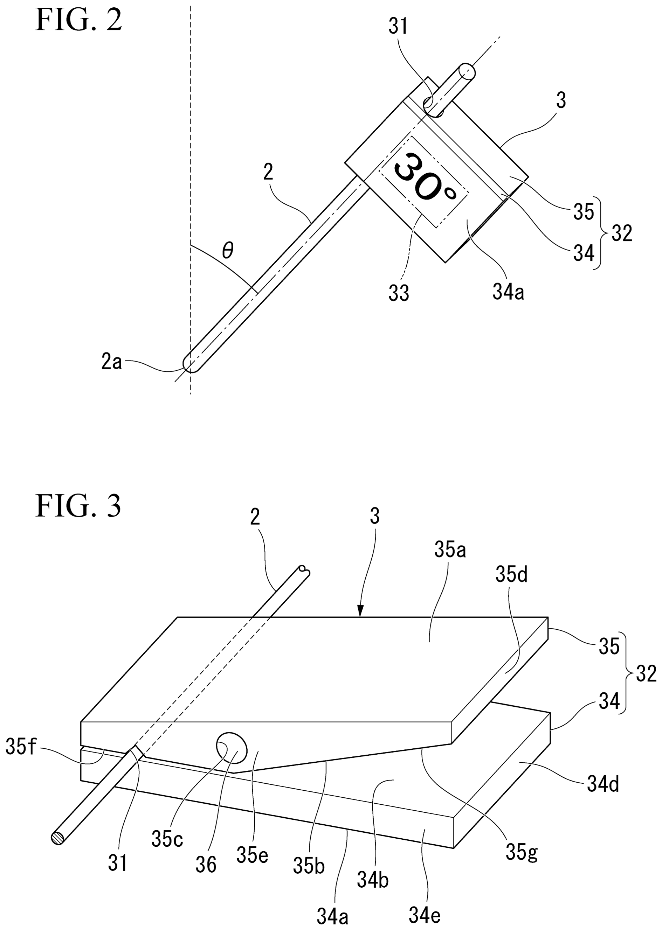

As shown in FIGS. 2 and 3, the puncture assisting tool 3 is attached to the puncture needle 2 to present, to a puncture practitioner, a tilt angle (tilt angle θ) of the puncture needle 2 from a vertical axis or a horizontal plane. The puncture assisting tool 3 includes a groove portion 31 (holding portion) that holds the puncture needle 2, a sensor unit 32 that can measure the tilt angle θ of the held puncture needle 2 from the vertical axis or the horizontal plane, and a tilt angle presentation unit 33 that presents the tilt angle θ of the puncture needle 2. Since the puncture assisting tool 3 is used by being attached to the puncture needle 2, it is preferable to perform sterilization during use.

As shown in FIG. 3, the sensor unit 32 of the puncture assisting tool 3 includes a fixed portion 34 and a movable portion 35 movably fixed to the fixed portion 34.

Hereinafter, an example will be described in which the puncture needle 2 is fixed by the fixed portion 34 and the movable portion 35 of the puncture assisting tool 3, but the method for fixing the puncture assisting tool 3 of the puncture needle 2 is not limited thereto. The fixation may be implemented using various methods such as attaching the puncture needle 2 to a predetermined position of the puncture assisting tool 3. A position where the puncture assisting tool 3 is fixed to the puncture needle 2 is not limited to the puncture needle 2, and the puncture assisting tool 3 may be fixed to a member extending from the puncture needle 2.

The fixed portion 34 is formed in a rectangular plate shape in a plan view, and includes a three-axis acceleration sensor (not shown) therein. At the fixed portion 34, the tilt angle presentation unit 33 is provided at an outer surface 34a opposite to one surface (inner surface 34b) where the movable portion 35 is provided. A short side portion 34d of the fixed portion 34 extends in a direction along a length direction of the puncture needle 2 held by the groove portion 31 in the plan view. A long side portion 34e of the fixed portion 34 extends in a direction orthogonal to the groove portion 31 in the plan view. The groove portion 31 is formed such that a sensor axis of the acceleration sensor matches the length direction of the puncture needle 2 when the puncture needle is fixed. A movable portion rotation shaft 36 that rotatably supports the movable portion 35 is provided at an intermediate portion of the inner surface 34b of the fixed portion 34 in a long side direction. An axial direction of the movable portion rotation shaft 36 is a direction along the short side portion 34d.

As shown in FIG. 3, the movable portion 35 is formed in a substantially rectangular plate shape in the plan view. The movable portion 35 is disposed such that an inner surface 35b faces the inner surface 34b of the fixed portion 34. A planar shape of the movable portion 35 is substantially the same as that of the fixed portion 34. That is, a short side portion 35d of the movable portion 35 extends in the direction along the length direction of the puncture needle 2 held by the groove portion 31 in the plan view. A long side portion 35e of the movable portion 35 extends in the orthogonal direction along the long side portion 34e of the fixed portion 34 in the plan view.

A rotation support portion 35c at an intermediate portion of the inner surface 35b of the movable portion 35 in a long side direction is rotatably supported by and coupled to the movable portion rotation shaft 36 provided at the fixed portion 34. The inner surface 35b of the movable portion 35 has a clamping surface 35f on a distal side of the rotation support portion 35c and a proximal surface 35g on a proximal side. The clamping surface 35f is tilted to approach an outer surface 35a from the rotation support portion 35c toward the distal side. The proximal surface 35g is tilted to approach the outer surface 35a from the rotation support portion 35c toward the proximal side.

The groove portion 31 having a V cross section is formed at the clamping surface 35f of the movable portion 35.

When the long side portion 34e of the puncture assisting tool 3 is also aligned with a vertical laser plane A to be described later or when a scale 380 to be described later is formed, a surface of the long side portion 34e of the fixed portion 34 and a surface of the long side portion 35e of the movable portion 35 are perpendicular to the length direction of the puncture needle 2 when the puncture needle 2 is locked. At this time, by locking the puncture needle 2 along a groove direction of the groove portion 31, an orientation of the length direction of the puncture needle 2 is determined in a direction orthogonal to the long side portion 35e of the movable portion 35. That is, the orientation of the puncture needle 2 is determined in a direction orthogonal to the long side portion 34e of the fixed portion 34.

Preferably, the groove portion 31, the fixed portion 34, and the movable portion 35 may be formed such that the length direction of the puncture needle 2 is parallel to a surface of the short side portion 34d of the fixed portion 34 and a surface of the short side portion 35d of the movable portion 35.

The movable portion 35 is rotatable to swing about the movable portion rotation shaft 36. A distal end of the movable portion 35 on the groove portion 31 side is biased in a direction approaching the inner surface 34b of the fixed portion 34 by a biasing force of a biasing member (not shown) such as a spring member. By pushing the proximal side of the movable portion 35 in a direction approaching the fixed portion 34 against the biasing force of the biasing member, the proximal surface 35g approaches the inner surface 34b of the fixed portion 34, and the clamping surface 35f is separated from the inner surface 34b of the fixed portion 34. In the groove portion 31, the puncture needle 2 can be held in a state of being interposed between the groove portion 31 and the inner surface 34b of the fixed portion 34. The groove portion 31 is set to have a groove shape and a groove depth that can press the puncture needle 2 when the puncture needle 2 is interposed between the groove portion 31 and the inner surface 34b of the fixed portion 34. Therefore, the groove shape of the groove portion 31 is not limited to the V groove as in the embodiment.

In this way, the movable portion rotation shaft 36, the biasing member, and the groove portion 31 in such a groove portion 31 correspond to a lock mechanism that clamps and fixes the puncture needle 2.

As shown in FIG. 2, the tilt angle presentation unit 33 is electrically connected to the acceleration sensor accommodated in the sensor unit 32, and numerically (digitally) displays the tilt angle θ of the puncture needle 2, for example. A practitioner using the puncture assisting tool 3 can recognize the angle of the puncture needle 2 held by the puncture assisting tool 3 by visually observing the tilt angle presentation unit 33 of the fixed portion 34.

A display format of the tilt angle θ of the tilt angle presentation unit 33 is not limited to a digital display, and may be a display of a symbol, a color, or the like that enables the practitioner to visually recognize the tilt angle θ. Sound, vibration, or the like may also be used. In the digital display, only a numerical value of the tilt angle θ may be simply displayed, or a unit "°" or a symbol may be added to the display as in the embodiment. A deviation from a set angle may be displayed, and for example, when the deviation from the set angle is equal to or larger than a predetermined threshold value, a special display (blinking, sound, or vibration) may be performed.

A display position of the tilt angle presentation unit 33 is not limited to the outer surface 34a of the fixed portion 34. For example, the tilt angle presentation unit 33 may be provided at the outer surface 35a of the movable portion 35, or the tilt angle presentation unit 33 may be provided on both the outer surface 34a of the fixed portion 34 and the outer surface 35a of the movable portion 35.

As the sensor in the fixed portion 34, the three-axis acceleration sensor is adopted as an example. The sensor unit 32 is set to measure the tilt angle θ with respect to the groove direction of the groove portion 31, that is, a vertical direction or a horizontal direction (the vertical direction in the embodiment) of the puncture needle 2 held by the groove portion 31 of the puncture assisting tool 3. As long as three axes can be detected, the sensor is not limited to the acceleration sensor, and any sensor may be used.

The sensor accommodated in the sensor unit 32 is not limited to the three-axis acceleration sensor, and for example, a two-axis acceleration sensor may be used, and the case of the two-axis acceleration sensor will be described in another embodiment (second embodiment) to be described later since there is a requirement different from that in the embodiment.

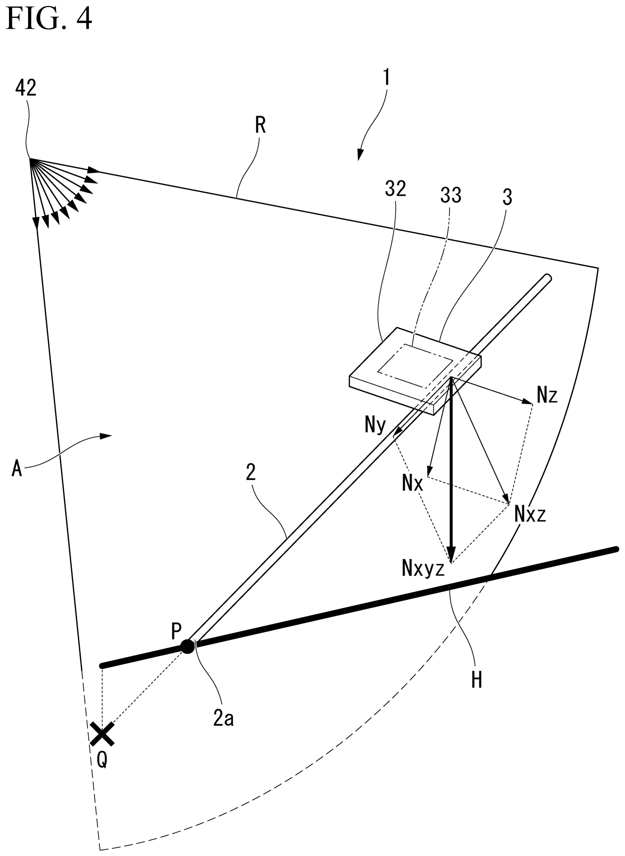

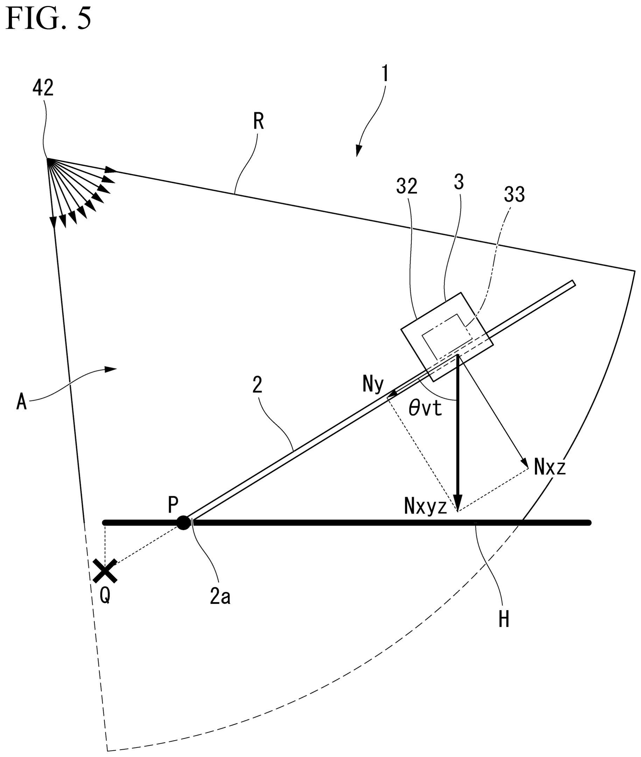

FIG. 4 is a perspective view showing a positional relationship between the puncture needle 2 and the puncture assisting tool 3 during a puncture. FIG. 5 is a side view of FIG. 4. The laser light R shown in FIGS. 4 and 5 is emitted from a laser irradiation unit 42 (to be described later) of the body surface-irradiating laser mechanism 4. An Ny axis shown in FIGS. 4 and 5 is an axis (first sensor axis) in the length direction of the puncture needle 2 (puncture needle direction), an Nx axis is a first orthogonal axis (second sensor axis) orthogonal to the axis (Ny axis) in the puncture needle direction, and an Nz axis is a second orthogonal axis (third sensor axis) orthogonal to both the axis (Ny axis) in the puncture needle direction and the first orthogonal axis (Nx axis). Here, a reference sign H in FIGS. 4 and 5 indicates a body surface marker line obtained by planar projection of a straight line connecting the puncture insertion point P and the puncture target point Q onto the body surface.

In the sensor unit 32, the first sensor axis Ny is set in the puncture needle direction of the puncture needle 2, the second sensor axis Nx is set in a direction at a right angle to the puncture needle 2, and the third sensor axis Nz is set in a direction at a right angle to both the axis Nx and the axis Ny. The tilt angle presentation unit 33 of the puncture assisting tool 3 holds the puncture needle 2 together with the puncture assisting tool 3 such that the laser light R displaying the vertical laser plane A including the puncture insertion point P and the puncture target point Q is emitted to the puncture needle 2, and presents, to the practitioner, the tilt angle θ of the puncture needle 2 from the vertical axis or the horizontal plane in a vertical direction Nxyz. For example, as shown in FIG. 5, when an angle θvt between the first sensor axis Ny and the vertical direction Nxyz is arctan(Nzz/Ny), the tilt angle θ of the puncture needle 2 from the horizontal plane in the plane of the vertical laser plane A is calculated by an expression of 90 - θvt and displayed on the tilt angle presentation unit 33.

After a puncture line along which the puncture needle 2 does not damage an important organ or the like is determined by the puncture navigation system 5 (see FIGS. 8 and 9) to be described later, the laser light R is emitted along the puncture line including the puncture target point Q and the puncture insertion point P, and the body surface marker line H (a line formed by a plane including the puncture insertion point P and the puncture target point Q in the body of the patient M at the body surface Ma of the patient M) is displayed. The practitioner can safely reach the puncture target point Q by aligning a tip 2a of the puncture needle 2 with the puncture insertion point P, aligning the length direction of the puncture needle 2 with the laser light R (a state in which line light formed by the laser light R is displayed on the puncture needle direction of the puncture needle 2), and holding and puncturing such that the value on the tilt angle presentation unit 33 is the set angle.

[Body Surface-Irradiating Laser Mechanism]

As shown in FIG. 6, the body surface-irradiating laser mechanism 4 includes a holding member 41 provided at the medical table 40 that supports the patient M, the laser irradiation unit 42 that is attached via the holding member 41 and emits the laser light R forming the line (body surface marker line H) formed at the body surface Ma of the patient M by the vertical laser plane A including the puncture insertion point P and the puncture target point Q in the body of the patient M, and a movement mechanism 43 that moves the laser irradiation unit 42 provided at the holding member 41.

The laser irradiation unit 42 forms the vertical laser plane A by emitting the planar laser light R downward. The laser irradiation unit 42 is provided above the patient M lying at the medical table 40. In the embodiment, the medical table 40 is preferably movable forward and backward relative to the inside of a CT gantry (not shown) or an MRI gantry, and in a case where the puncture target point Q is determined by ultrasound or the like, such a configuration may not be provided. The laser irradiation unit 42 is preferably provided or moved on an opposite side of the practitioner and the CT gantry such that a puncture procedure of the practitioner is not interfered with. Depending on a situation, the laser irradiation unit 42 may be provided or moved on the same side as the practitioner.

The holding member 41 includes, as an example, leg frames 411 detachably erected from left and right ends 40a and 40b of the medical table 40, and a horizontal frame 412 that connects upper ends of the leg frames 411. The holding member 41 detaches the leg frames 411 from the medical table 40 when the patient M gets on or off the medical table 40, and attaches the leg frames 411 after the patient M gets on the medical table 40. A position where the holding member 41 is attached to the medical table 40 can be changed as desired.

In FIG. 6, the leg frames 411 are provided on both sides such that the body surface-irradiating laser mechanism 4 straddles the patient M, and alternatively, the leg frame 411 may be provided on one side and the body surface-irradiating laser mechanism 4 may be held by the leg frame 411 on the one side. In this case, the body surface-irradiating laser mechanism 4 may be pivotable with the leg frame 411 serving as a pivot, may be oriented in the direction of the body axis O before the patient M gets on the medical table 40, and may be pivoted to above a predetermined position of the patient M after the patient M gets on the medical table 40 and be fixed at the position using a fixing mechanism (not shown).

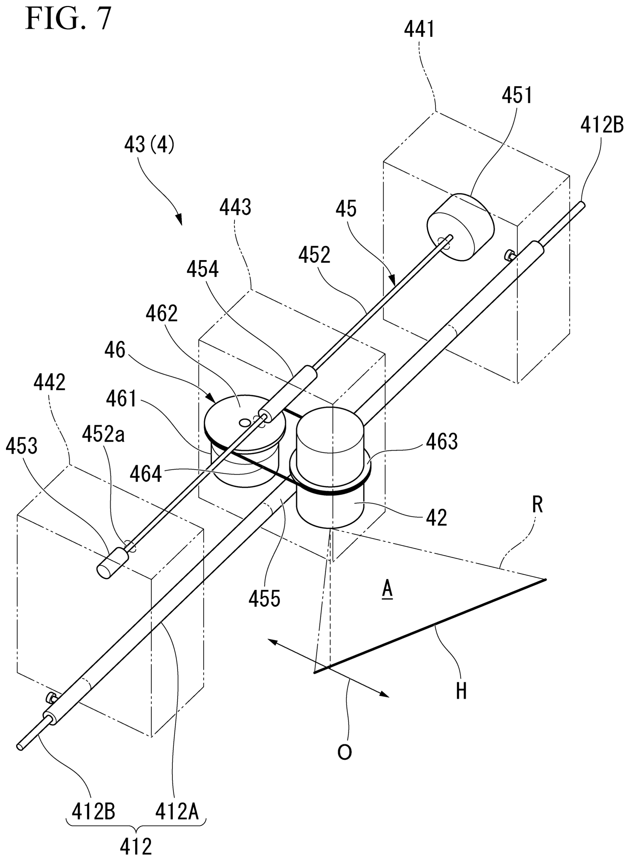

Each leg frame 411 is provided with auxiliary legs 413 branched downward. The leg frame 411 can expand and contract, and thus has an adjustable length. That is, the leg frame 411 includes a large-diameter tube 411A and a small-diameter tube 411B that is inserted into an upper end of the large-diameter tube 411A and can expand and contract, and a height of the horizontal frame 412 can be set as desired by adjusting to any length and fixing. The horizontal frame 412 can expand and contract, and thus has an adjustable length. That is, as shown in FIG. 7, the horizontal frame 412 includes a large-diameter tube 412A and small-diameter tubes 412B that are inserted into both ends of the large-diameter tube 412A and can expand and contract, and can be set to a length corresponding to a width dimension of the medical table 40 by adjusting to any length and fixing. Cross-sectional shapes of the leg frame 411 and the horizontal frame 412 may be circular or rectangular and are not particularly limited.

The movement mechanism 43 includes a rotation movement mechanism 46 that holds the laser irradiation unit 42 rotatably around the vertical axis, and a linear movement mechanism 45 that holds the laser irradiation unit 42 to allow movement thereof in a linear direction along the horizontal frame 412.

As shown in FIG. 7, the movement mechanism 43 includes a first fixed case 441 and a second fixed case 442 immovably provided on both sides in a length direction of the horizontal frame 412, and a moving case 443 movably provided along the horizontal frame 412 between the first fixed case 441 and the second fixed case 442 in the length direction of the horizontal frame 412. The laser irradiation unit 42 is accommodated in the moving case 443 such that the laser light R can be emitted downward.

The linear movement mechanism 45 will be described. The first fixed case 441 accommodates a linear drive unit 451 such as a stepping motor having a horizontal axis as a rotation center, and is connected to a feed screw 452 to which rotation of the linear drive unit 451 is transmitted. The feed screw 452 extends parallel to the extending direction of the horizontal frame 412, and a screw tip 452a is rotatably supported by a bearing 453 accommodated in the second fixed case 442.

The moving case 443 accommodates a feed screw nut 454 that is fitted to the feed screw 452 and moves along the feed screw 452, and a fitting sliding sleeve 455 that fits and slides along the horizontal frame 412. The feed screw nut 454 moves the moving case 443 along the horizontal frame 412 together with the laser irradiation unit 42. Accordingly, the laser irradiation unit 42 moves in a direction at a right angle to the body axis O of the patient M.

The rotation movement mechanism 46 will be described. The moving case 443 accommodates a rotation drive unit 461 such as a stepping motor having the vertical axis as a rotation center. The rotation movement mechanism 46 is configured such that a first pulley 462 is pivotally supported by the rotation drive unit 461, a second pulley 463 is pivotally supported by the laser irradiation unit 42, and rotation of the rotation drive unit 461 is transmitted to the laser irradiation unit 42 by transmission with a connecting belt 464 that connects the first pulley 462 and the second pulley 463.

As shown in FIG. 6, a horizontal movement operation and a rotation operation of the laser irradiation unit 42 in the body surface-irradiating laser mechanism 4 and an on-off operation of the laser light R are controlled in a wired or wireless manner using an operation panel 47.

An overall size of the body surface-irradiating laser mechanism 4 is preferably a size that allows the entire body surface-irradiating laser mechanism 4 to pass without interfering with a gantry of CT or the like when the medical table 40 is moved in a state in which the body surface-irradiating laser mechanism 4 is set at the medical table 40.

Configurations, dimensions, shapes, and the like of the holding member 41, the linear movement mechanism 45, and the rotation movement mechanism 46 in the body surface-irradiating laser mechanism 4 in FIGS. 6 and 7 are examples, and can be appropriately changed. For example, the linear drive unit 451 and the rotation drive unit 461 are not limited to stepping motors, and may be general motors or other components that can be mechanically driven. A driving force transmitting unit is not limited to the pulley and the belt, and alternatively, may be a chain, a gear, a direct drive, or another unit for transmitting a driving force.

The body surface-irradiating laser mechanism 4 is preferably provided separately from line laser provided at a gantry in related art as shown in FIGS. 6 and 7, but is not limited thereto. Body axis laser or line laser provided at the gantry may have a function of the laser irradiation unit in the form of a movement mechanism or a rotation movement mechanism. The body axis laser provided in a general gantry is fixed such that a laser plane matches the body axis O, and the line laser is fixed such that a line laser plane is perpendicular to the body axis O. The body axis laser or the line laser may be movable and rotatable to serve as the body surface-irradiating laser mechanism in the invention.

The body axis laser and the line laser provided at the conventional gantry are used as a reference of the body axis O and a reference of CT imaging, and a configuration in which the body surface-irradiating laser mechanism 4 is used in combination as shown in FIGS. 6 and 7 is preferable from the viewpoint of accuracy, versatility, and the like.

In the embodiment, it is preferable that the laser irradiation unit 42 forms the vertical laser plane A for vertically emitting laser as a laser plane that simplifies calculation and is clear and intuitively recognizable by the practitioner.

However, depending on the body surface marker line H to the patient M, the laser irradiation unit 42 is not limited to vertically emitting the laser, and may have a laser plane at a predetermined angle.

The linear movement mechanism 45 that moves in a direction orthogonal to the body axis O is shown in FIG. 6 and the like, and alternatively, the linear movement mechanism 45 can be moved in a direction other than the direction orthogonal to the body axis O, and such a configuration may be adopted.

[Puncture Navigation System]

The puncture navigation system 5 specifies the puncture target point Q in the body of the patient M from a multi planar reconstruction (CT-MPR) image, an MRI image, or an ultrasound image. As shown in FIGS. 8 and 9, among insertion lines along which the puncture needle 2 can reach the puncture target point Q, an insertion line that can avoid important organs and blood vessels is obtained. In a case where there are a plurality of insertion lines, a calculation unit (not shown) is provided to determine the puncture insertion point P by selecting a most safe insertion line having a short insertion length, to determine the plane including the puncture target point Q and the puncture insertion point P, the puncture insertion point P where the puncture needle 2 is inserted, an insertion angle, and an insertion length, and to calculate an angle from the body axis O to the plane. The insertion angle, the insertion length, and the angle from the body axis O to the plane may be read and determined from the CT-MPR image. An insertion angle error, an insertion length error, and the like allowable in each insertion line may also be calculated, and a degree of insertion safety may be calculated based on a predetermined determination criterion. The puncture navigation system 5 also includes a control unit (not shown) that moves the laser irradiation unit 42 to the vertical laser plane A using the movement mechanism 43 as shown in FIG. 6 based on determination and calculation by the calculation unit. The calculation unit and the control unit include a known electronic circuit unit or the like including a CPU (not shown), a storage unit including storage elements such as a ROM and a RAM, and an interface circuit or the like. The puncture navigation system 5 uses one point among coordinates of the puncture target point Q (target point coordinates T) and coordinates of a plurality of puncture insertion points P (insertion point coordinates S) that are determined and calculated, and automatically sets a rotation angle θh of the laser irradiation unit 42 and an X-axis direction position Xj by the control unit.

As shown in FIG. 8, in a method for calibrating the laser irradiation unit 42, first, a rotation angle of the laser light R from the laser irradiation unit 42 is set to 0 degrees (at a right angle to CT line laser Rc, in other words, in a direction along the body axis O), for example, according to laser Ro of the body axis O (usually in a longitudinal direction of a bed center) provided at CT, and an X coordinate thereof is set to Xc (FIG. 8 shows a state in which laser light (laser light R) emitted to the body axis O by the laser irradiation unit 42 and the laser Ro emitted along the body axis O provided at the CT or the like overlap each other). Then, the laser light R is rotated at a rotation angle of 90 degrees (in parallel to a CT line laser Zct) to be parallel to the CT line laser Rc. At this time, an interval Zjct between the laser of the laser irradiation unit 42 and the CT line laser Rc is measured to obtain Zj.

As shown in FIG. 9, specifically, as a method of aligning the laser light R of the laser irradiation unit 42 with the insertion point coordinates S and the target point coordinates T, inserting the puncture needle 2 from the insertion point coordinates S, and causing the tip of the puncture needle 2 to reach the target point coordinates T when the patient M is in a supine position, two methods will be described as examples using CT.

As a first method, the coordinates S of the puncture insertion point P (hereinafter, referred to as insertion point coordinates S) and the coordinates T of the puncture target point Q (hereinafter, referred to as target point coordinates T) are determined from the CT-MPR image, insertion point coordinates S(Xs, Ys, Zs) and target point coordinates T(Xt, Yt, Zt) are read, the rotation angle θh of the vertical laser plane A including the insertion point coordinates S and the target point coordinates T from the body axis, and the X coordinate Xj of an intersection of the vertical laser plane A with Zj are calculated, and the laser irradiation unit 42 is moved.

Further, an inclination angle θv of a line segment ST in the vertical laser plane A from the horizontal plane and a distance Dst corresponding to the puncture depth between the insertion point coordinates S and the target point coordinates T are calculated, the needle tip of the puncture needle 2 is placed at the puncture point coordinates S, the puncture needle 2 is placed in the vertical laser plane A such that the laser light R is emitted to the entire puncture needle 2, the puncture needle 2 is tilted such that an angle of the tilt angle presentation unit 33 is θv, and the puncture needle 2 is advanced by the depth Dst, whereby the tip of the puncture needle 2 reaches the target point coordinates T.

The above θh, Xj (one of four equations), θv, and Dst can be calculated by the following Equations (1) and (1').

$$\theta h = \arctan\{(Xt - Xs)/(Zt - Zs)$$

$$Xj = Xs - (Zs - Zj) \times \tan(\theta h)$$

$$Xj = Xt - (Zt - Zj) \times \tan(\theta h)$$

$$Xj = Xs - (Xs - Xt) \times (Zs - Zj)/(Zs - Zt)$$

$$Xj = Xt - (Xs - Xt) \times (Zt - Zj)/(Zs - Zt)$$

$$Dxz = \sqrt{\{(Xt - Xs)^2 + (Zt - Zs)^2\}}$$

$$\theta v = \arctan\{(Yt - Ys)/Dxz\} \dots (1)$$

$$Dst = \sqrt{\{(Xt - Xs)^2 + (Yt - Ys)^2 + (Zt - Zs)^2)\}} \dots (1')$$

As a second method that is another method, there is a method for calculating only Xj without calculating θh, θv, and Dst as described above.

A CT-MPR sagittal plane is rotated about the vertical axis, a sagittal plane including the insertion point coordinates S and the target point coordinates T is determined, the rotation angle θh of the sagittal plane and the insertion point coordinates (Xs, Ys, Zs) or the target point coordinates (Xt, Yt, Zt) are read, the X coordinate Xj of the intersection of the vertical laser plane A with Zj is calculated based on the rotation angle θh of the sagittal plane and the insertion point coordinates S or the target point coordinates T, and the laser irradiation unit 42 is moved.

Further, the inclination angle θv of the line segment ST in the sagittal plane from the horizontal plane and the distance Dst corresponding to the puncture depth between the insertion point coordinates S and the target point coordinates T are read, the needle tip of the puncture needle 2 is placed at the puncture point coordinates S, the puncture needle 2 is placed in the vertical laser plane A such that the laser light R is emitted to the entire puncture needle 2, the puncture needle 2 is tilted such that an angle of the tilt angle presentation unit 33 is θv, and the puncture needle 2 is advanced by the depth Dst, whereby the tip of the puncture needle 2 can reach the target point coordinates T.

In any of the above methods, or combination of both methods, the tip of the puncture needle 2 can reach the target point coordinates T using the laser light R of the laser irradiation unit 42 as a guide. The puncture medical procedure can be performed using any one of or a combination of the respective methods depending on a function of an apparatus of CT or the like to be used.

The puncture navigation system 5 may include a calibration unit that calibrates movement of the patient M during the medical procedure. During the medical procedure, the patient M may move and the laser light R of the laser irradiation unit 42 may deviate from the determined body surface marker line H including the puncture insertion point P.

In order to prevent this, the puncture system according to the invention may include a marking element.

For example, a marking element may be provided such that the determined puncture insertion point P or body surface marker line H can be visually recognized. For example, an attachable marking element or a drawable marking element may be provided at the puncture insertion point P or a surface of the body surface marker line H on the body surface Ma. Even when the laser light R of the laser irradiation unit 42 deviates from the body surface marker line H due to the movement of the patient M, the marking element can be used to visually readjust a position and rotation of the laser light R of the laser irradiation unit 42 to match the body surface marker line H.

The puncture navigation system 5 may also include an imaging unit such as a camera, detect the marking element, calculate a degree of deviation using an image analysis function, drive the body surface-irradiating laser mechanism 4 by the control unit based on the calculated degree, automatically control a linear direction and a rotation direction, and calibrate the laser light R from the laser irradiation unit 42 such that the laser light R is always aligned with the body surface marker line H without deviation.

[Medical Procedure Steps Using Puncture System]

Next, operation steps of puncturing the puncture target point Q of the patient M with the puncture needle 2 at an accurate angle using the puncture assisting tool 3 will be described in detail.

First, as shown in FIG. 1, the puncture target point Q indicating a position of a target such as a malignant tumor is read from a CT image or the like, and the practitioner determines a puncture direction and the puncture insertion point P that enables a safe puncture to the puncture target point Q in a preoperative plan. By this determination, a straight line connecting the puncture target point Q and the puncture insertion point P is determined, and further, a straight line (body surface marker line H) projected at the body surface Ma of the patient M in this straight line is determined. The tilt angle θ between an extension line of the straight line connecting the puncture target point Q and the puncture insertion point P and the vertical axis or the horizontal plane is calculated. The tilt angle θ is a puncture angle of the puncture needle 2. Then, the laser light R is emitted from the laser irradiation unit 42 to form the vertical laser plane A passing through the body surface marker line H. The operation so far is performed by the control unit of the puncture navigation system 5 described above in the embodiment.

Specifically, as shown in FIG. 6, the control unit controls the linear movement mechanism 45 and the rotation movement mechanism 46 of the body surface-irradiating laser mechanism 4 set above the patient M, and performs control to calculate and adjust a position of the laser irradiation unit 42 in the horizontal direction orthogonal to the body axis O in a plan view and an angle in the rotation direction around the vertical axis. Whether the body surface marker line H is displayed at an accurate position by the laser irradiation unit 42 may be checked by imaging with an imaging unit and performing image analysis.

Even when the puncture navigation system 5 is not used, similarly, the practitioner or the like determines the puncture insertion point P using the CT image or the like, and the practitioner or the like adjusts the position of the laser irradiation unit 42 by operating the operation panel 47 such that the vertical laser plane A is formed by calculating the position in the horizontal direction orthogonal to the body axis O in the plan view of the laser irradiation unit 42 and the angle in the rotation direction around the vertical axis or by reading from the CT image or the like.

Next, the puncture using the puncture needle 2 is performed. First, the puncture assisting tool 3 is attached to the puncture needle 2. As shown in FIG. 3, the puncture needle 2 is held in the groove portion 31 of the movable portion 35 and interposed between the groove portion 31 and the fixed portion 34, whereby the sensor unit 32 including the sensor therein is attached to the puncture needle 2.

Thereafter, as shown in FIGS. 4 and 5, the practitioner grips the puncture needle 2 to which the sensor unit 32 is attached using a puncture holder or the like, and brings the tip 2a of the puncture needle 2 close to the puncture insertion point P at the body surface Ma such that the vertical laser plane A comes into contact with the entire puncture needle 2. Then, the puncture needle 2 is disposed in the plane of the vertical laser plane A, and the tilt angle θ of the puncture needle 2 is adjusted while checking the tilt angle presentation unit 33 of the puncture assisting tool 3. That is, the puncture needle 2 is inserted into the body at the predetermined tilt angle θ. During this puncture operation, the practitioner still checks the numerical value (tilt angle) displayed on the tilt angle presentation unit 33, and depending on a situation, the practitioner further repeatedly acquires a CT image or the like to check an insertion situation of the puncture needle 2 into the body.

[Effects]

As described above, the puncture assisting tool 3 according to the embodiment is detachably provided with the puncture needle 2 that punctures using the line (body surface marker line H) formed at the body surface Ma of the patient M by the laser plane including the puncture insertion point P and the puncture target point Q in the body of the patient M. The puncture assisting tool 3 includes the groove portion 31 (holding portion) that holds the puncture needle 2, the sensor unit 32 that can measure the angle of the held puncture needle 2 from the vertical axis or the horizontal plane, and the tilt angle presentation unit 33 that presents the angle.

In the puncture assisting tool 3 according to the embodiment, when the puncture needle 2 held by the groove portion 31 is directed at the predetermined tilt angle along the line formed at the body surface Ma of the patient M by the laser plane including the puncture insertion point P and the puncture target point Q in the body of the patient M, the angle of the puncture needle 2 from the vertical axis or the horizontal plane is measured by the sensor unit 32, and the tilt angle that is the measured value can be presented by the tilt angle presentation unit 33. Therefore, the practitioner can accurately check the tilt angle θ of the puncture needle 2 in real time by viewing the tilt angle presentation unit 33, and can safely reach the puncture target point Q only by accurately puncturing the inside of the body of the patient M while adjusting the tilt angle of the puncture needle 2.

For example, in a CT-guided puncture, when the puncture is performed in a CT cross section, the tilt angle of the puncture needle 2 with respect to the vertical axis or the horizontal plane can be presented to the practitioner during the puncture from when the puncture is started.

Therefore, in the embodiment, the number of times of adjustment of the tilt angle of the puncture needle 2 during the puncture can be reduced, and a time for the medical procedure can be shortened. Therefore, in the case of the CT-guided puncture, X-ray exposure of the practitioner and the patient M can be reduced. Further, in order to reduce the X-ray exposure, even when the practitioner holds the puncture needle 2 with the puncture holder or the like and performs CT imaging, the puncture needle tilt angle is always presented to the practitioner by the tilt angle presentation unit 33, and thus the tilt angle of the puncture needle 2 can be prevented from fluctuating.

In the embodiment, when the puncture needle tilt angle is finely adjusted during the puncture, the puncture needle tilt angle is always presented, and thus the puncture tilt angle can be finely adjusted based on a specific angle numerical value. Even when the practitioner is inexperienced, it is possible to receive an instruction on the puncture angle with a specific angle numerical value from a medical procedure instructor, and the inexperienced practitioner can finely adjust the puncture needle tilt angle while referring to angle presentation on the tilt angle presentation unit 33.

In the puncture assisting tool 3 according to the present embodiment, the holding portion includes the groove portion 31, and the fixing mechanism that holds and fixes the puncture needle 2 in the groove portion 31 by the fixed portion 34 and the movable portion 35 is provided.

In the embodiment, the sensor unit 32 for presenting the tilt angle of the puncture needle 2 has a structure that can be interposed and fixed between the fixed portion 34 and the movable portion 35, and the sensor unit 32 can be easily attached to or detached from the existing puncture needle 2. Therefore, the sensor unit can be easily attached to the puncture needle 2 for puncturing in a short time. Further, even when a plurality of punctures are required in one operation, since the sensor unit 32 can be removed and easily attached to another puncture needle 2 after the puncture, the plurality of punctures can be performed efficiently and cleanly at low cost.

The body surface-irradiating laser mechanism 4 according to the embodiment includes the holding member 41 provided at the medical table 40 that supports the patient M, the laser irradiation unit 42 that is attached via the holding member 41 and emits the laser along the line formed at the body surface Ma of the patient M by the laser plane including the puncture insertion point P and the puncture target point Q in the body of the patient M, and the movement mechanism 43 that moves the laser irradiation unit 42 provided at the holding member 41.

In the embodiment, the laser irradiation unit 42 different from CT laser can be provided, and the laser irradiation unit 42 can emit the laser forming the vertical laser plane A including the puncture insertion point P and the puncture target point Q in the body of the patient M. Therefore, the practitioner can direct the orientation of the puncture needle 2 along the line (body surface marker line H) formed at the body surface Ma in the plan view by aligning the puncture needle 2 including the sensor unit 32 with the vertical laser plane A, and can perform the puncture by adjusting the tilt angle θ of the puncture needle 2 while checking the presentation on the tilt angle presentation unit 33 in a state in which the puncture needle 2 is placed within the vertical laser plane A.

In the embodiment, since the laser irradiation unit 42 provided at the holding member 41 can be moved to a predetermined position by the movement mechanism 43, a degree of freedom is extremely high, the laser can be displayed at the position of the body surface marker line H in a wide range on the body surface at any place, the vertical laser plane A irradiated by the laser irradiation unit 42 can be displayed at any position, and the puncture medical procedure can be easily and accurately performed according to the displayed laser.

Since the laser irradiation unit 42 does not need to be held by a hand of a person such as an assistant, there is no hand shaking. Since the laser irradiation unit 42 does not come into contact with the patient, the laser can be stably displayed.

In the body surface-irradiating laser mechanism 4 according to the embodiment, the movement mechanism 43 includes the rotation movement mechanism 46 that rotatably holds the laser irradiation unit 42.

In this case, since the laser irradiation unit 42 can be adjusted in the rotation direction around the vertical axis by the rotation movement mechanism 46, a display position can have a higher degree of freedom, and the vertical laser plane A intersecting a CT cross section can be formed with high accuracy.

Further, in the body surface-irradiating laser mechanism 4 according to the embodiment, the movement mechanism 43 includes the linear movement mechanism 45 that movably holds the laser irradiation unit 42 in the linear direction.

In this case, since the laser irradiation unit 42 can be adjusted in the linear direction orthogonal to the body axis O of the patient M by the linear movement mechanism 45, the vertical laser plane A and the body surface marker line H can be accurately formed.

In the puncture navigation system 5 according to the embodiment, the puncture insertion point P and the puncture target point Q in the body of the patient M are determined, and the laser plane including the puncture target point Q and the puncture insertion point P, the insertion angle at which the puncture needle 2 is inserted, the insertion length, and the angle from the body axis O to the laser plane can be accurately and automatically calculated by the control unit. Therefore, since efficiency of a puncture operation can be improved and angle calibration can be easily performed in a short time, for example, even when a position of the patient M changes during the puncture, the puncture operation can be performed while adjusting the puncture needle 2 based on the angle calculated by the puncture navigation system 5.

In the puncture system 1 according to the embodiment, the control unit can move the laser irradiation unit 42 to the vertical laser plane A using the movement mechanism 43.

According to the puncture system 1, the puncture assisting tool 3, the body surface-irradiating laser mechanism 4, and the puncture navigation system 5 according to the embodiment, the tilt angle of the puncture needle 2 punctured into the patient M can be easily and accurately checked, and the puncture can be performed with high accuracy.

[Others]

Next, a puncture system, a puncture assisting tool, a body surface-irradiating laser mechanism, and a puncture navigation system according to other embodiments will be described with reference to the accompanying drawings. Members and portions that are the same as or similar to those in the first embodiment are denoted by the same reference signs, descriptions thereof are omitted, and configurations different from those of the first embodiment will be described.

[Second Embodiment]

The three-axis acceleration sensor is used in FIGS. 4 and 5, and alternatively, a two-axis acceleration sensor may be used. In this case, the sensor unit including the two-axis acceleration sensor can be implemented by setting the first sensor axis Ny in the puncture needle direction of the puncture needle 2 and setting the second sensor axis Nx in a direction at a right angle to the puncture needle 2. At this time, the tilt angle θ of the puncture needle 2 measured by the sensor unit 32 is displayed on the tilt angle presentation unit 33 of the puncture assisting tool. The puncture assisting tool 3 holds the puncture assisting tool 3 such that the laser light R displaying the vertical laser plane A including the puncture insertion point P and the puncture target point Q is emitted to the long side portion 34e of the fixed portion 34 perpendicular to the length direction of the puncture needle 2 in FIG. 3 and the short side portion 34d of the fixed portion 34 extending in the direction along the length direction of the puncture needle 2, and presents, to the practitioner, the tilt angle θ of the puncture assisting tool 3 from the vertical axis or the horizontal plane in a vertical direction Nxy. A two-axis acceleration sensor scale may be provided such that the laser is easily aligned with the long side portion 34e and the short side portion 34d to which the laser light R is emitted.

[Third Embodiment]

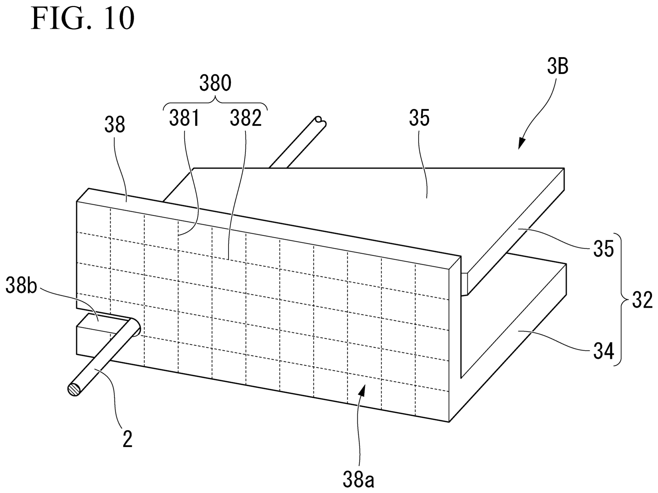

Next, a puncture assisting tool 3B according to a third embodiment will be specifically described with reference to FIGS. 10 to 13.

There is a method of aligning the vertical laser plane A emitted by the laser irradiation unit 42 (see FIG. 1) with the puncture needle 2 as described above, and alternatively as shown in FIG. 10, there is a method of aligning the scale 380 of a perpendicular surface 38a to be described later with a CT cross section Ac serving as the vertical laser plane A in the puncture assisting tool 3B of the third embodiment.

In the puncture assisting tool 3B, a perpendicular wall 38 having the perpendicular surface 38a perpendicular to the puncture needle 2 is fixed to the fixed portion 34. The perpendicular wall 38 is fixed such that the perpendicular surface 38a is parallel to one long side portion of the fixed portion 34. The fixed portion 34 and the perpendicular wall 38 are formed in an L shape in a plan view of a side surface. The perpendicular wall 38 is formed with a notch 38b which penetrates the perpendicular wall 38 in a thickness direction and into which the puncture needle 2 can be inserted from an outer peripheral edge of the perpendicular wall 38.

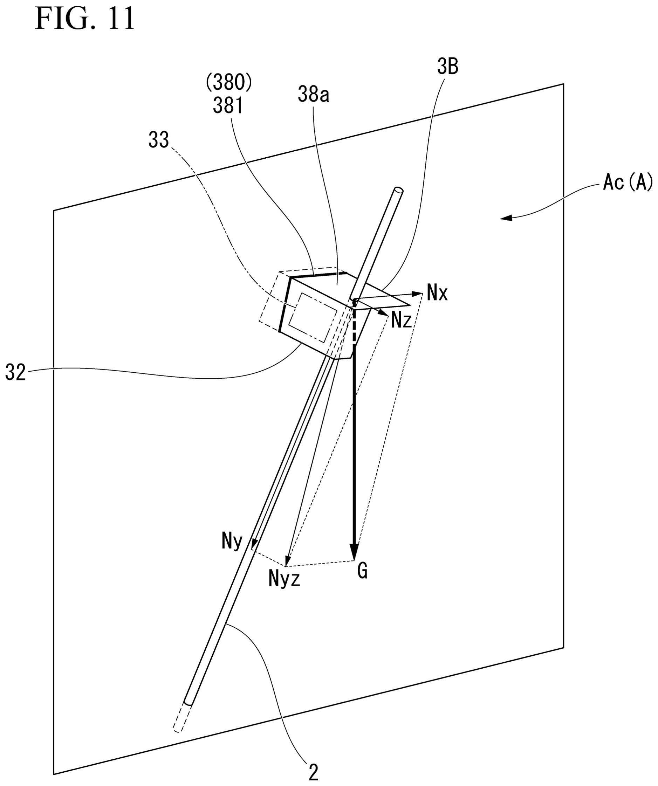

The scale 380 is provided at the perpendicular surface 38a of the perpendicular wall 38. The scale 380 includes a vertical-plane-parallel scale 381 for aligning the Nx axis shown in FIGS. 11 to 13 in parallel to the vertical laser plane A (CT cross section Ac), and a deviation angle scale 382 provided perpendicular to the vertical-plane-parallel scale 381. A plurality of vertical-plane-parallel scales 381 are displayed at regular intervals at the perpendicular surface 38a. A plurality of deviation angle scales 382 are displayed at regular intervals at the perpendicular surface 38a. The respective intervals between the vertical-plane-parallel scales 381 and the deviation angle scales 382 are set to any intervals.

The display on the perpendicular surface 38a is not limited to the scale 380 that is line marking as shown in FIG. 10, and may be, for example, a display where square patterns arranged at predetermined intervals function as the scale or a display of a scale having a wide width. In addition, the intervals may not be equal intervals, and may be intervals in consideration of an allowable error.

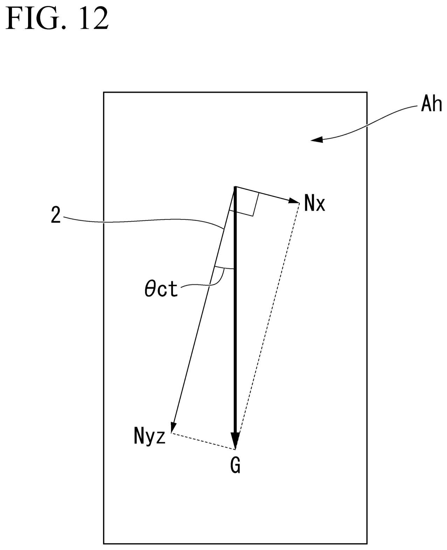

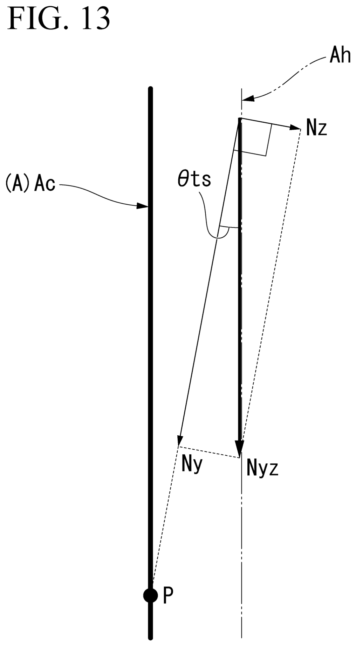

FIG. 11 is a perspective view showing a positional relationship between the puncture needle 2 and the puncture assisting tool 3B during a puncture. FIG. 12 shows a vertical plane Ah parallel to the vertical laser plane A (CT cross section Ac) including the Nx axis in FIG. 11. FIG. 13 shows an Ny-axis-Nz-axis plane in FIG. 11. The CT line laser Rc or the laser light R from the laser irradiation unit 42 shown in FIGS. 11 to 13 is emitted to the scale 380 and the tip of the puncture needle 2. The Ny axis shown in FIGS. 11 to 13 is an axis (first sensor axis) in the length direction of the puncture needle 2 (puncture needle direction), the Nx axis is the first orthogonal axis (second sensor axis) orthogonal to the axis (Ny axis) in the puncture needle direction in the vertical plane Ah parallel to the vertical laser plane A, and the Nz axis is the second orthogonal axis (third sensor axis) orthogonal to both the axis (Ny axis) in the puncture needle direction and the first orthogonal axis (Nx axis). The puncture assisting tool 3B has the vertical-plane-parallel scale 381 of the perpendicular surface 38a for identifying parallelism between the Nx axis and the vertical laser plane A.

A three-axis acceleration sensor is used as the sensor unit 32 in the puncture assisting tool 3B in the third embodiment. In the sensor unit 32, the first sensor axis Ny is set in the puncture needle direction of the puncture needle 2, the second sensor axis Nx is set in the direction at a right angle to the puncture needle 2, and the third sensor axis Nz is set in the direction at a right angle to both the axis Nx and the axis Ny. At this time, the tilt angle θ of the puncture needle 2 measured by the sensor unit 32 is displayed on the tilt angle presentation unit 33 of the puncture assisting tool 3B. The puncture assisting tool 3B holds the sensor unit 32 while aligning the CT line laser Rc displaying the vertical laser plane A including the puncture insertion point P or the laser light R from the laser irradiation unit 42 with the vertical-plane-parallel scale 381 of the perpendicular surface 38a, and presents, on the tilt angle presentation unit 33, any one or both of the tilt angle θ between a vertical line and the puncture needle 2 projected at the vertical laser plane A and the tilt angle between the vertical laser plane A and the puncture needle 2.

For example, as shown in FIG. 12, a tilt angle θct between the vertical line and the puncture needle 2 projected within a plane of the vertical plane Ah including the Nx axis parallel to the vertical laser plane A is obtained by Equation (6).

$$\mathrm{Nyz} \;=\; \sqrt{\{(\mathrm{Ny})^2 \;+\; (\mathrm{Nz})^2\}}$$

$$\theta\mathrm{ct} \;=\; \arctan(\mathrm{Nx/Nyz})\ldots\;(6)$$

As shown in FIG. 13, a tilt angle θts between the vertical laser plane A and the puncture needle 2 is obtained by Equation (7).

$$\theta\mathrm{ts} \;=\; \arctan(\mathrm{Nz/Ny})\ldots\;(7)$$

When the CT cross section Ac is used as the vertical laser plane A, the tilt angle θct matches a puncture needle angle in a CT image, and θts matches arctan(Ds/Ls), which is easy to understand for the practitioner using CT. Here, Ls represents a puncture needle length projected at the CT image, and Ds represents a slice thickness of the CT image.

The tilt angle of the puncture needle 2 projected at the vertical laser plane A relative to the vertical axis or the horizontal plane and the tilt angle of the puncture needle 2 from the vertical laser plane A can be presented to the practitioner during the puncture from when the puncture is started.

Even when the puncture is performed outside the vertical laser plane A, the tilt angle of the puncture needle 2 projected at the CT line vertical laser plane A relative to the vertical axis or the horizontal plane and the tilt angle of the puncture needle 2 from the CT cross section can be presented to the practitioner during the puncture from when the puncture is started.

[Fourth Embodiment]

In the puncture assisting tool 3 shown in FIG. 2, the tilt angle presentation unit 33 may be separable from the sensor unit 32. The tilt angle presentation unit 33 is communicable with the sensor unit 32 in a wired or wireless manner, and displays the tilt angle θ of the puncture needle 2 measured by the sensor unit 32 (when a presentation method is sound, vibration, or the like, notification is performed). That is, the sensor unit 32 and the tilt angle presentation unit 33 are not limited to being integrally provided. In this case, only the sensor unit 32 may be sterilized, and the tilt angle presentation unit 33 may be used in a sterilized bag without being sterilized.

The tilt angle presentation unit 33 includes, for example, a display mode switching button and a switch, and can switch a state of the sensor unit 32 attached to the puncture needle 2 and the displayed tilt angle θ. An operation of the sensor unit 32 may be changeable by a switching button or a switch of the tilt angle presentation unit 33. A tablet terminal or the like may be used as the tilt angle presentation unit 33.

[Fifth Embodiment]

A puncture system 1B in a fifth embodiment shows an example of a case where the puncture target point Q in the body of the patient M, the puncture insertion point P, and the tilt angle θ of the puncture needle 2 are determined using, for example, CT, MRI, and an ultrasound probe without using the CT cross section Ac by CT cross section guide laser.

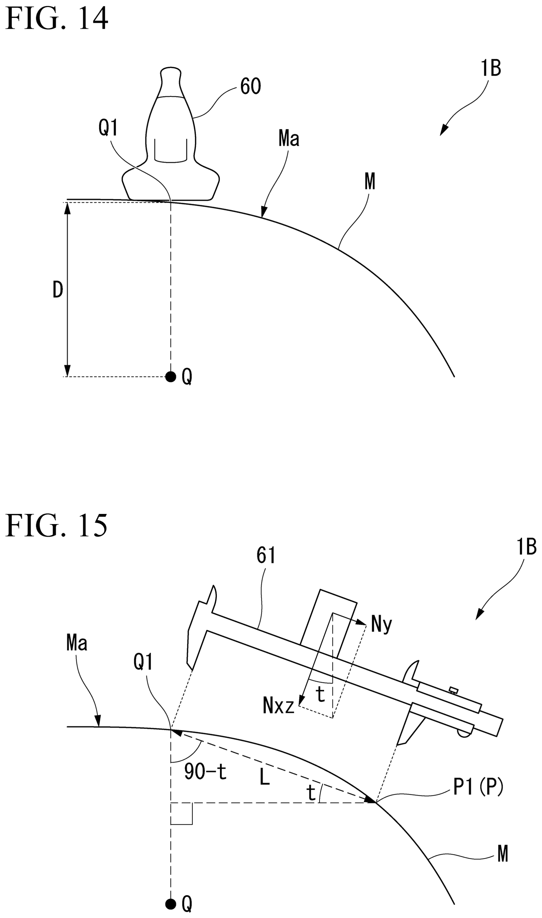

FIG. 14 shows an overview of the puncture system 1B using an ultrasound probe 60. As shown in FIG. 14, in the puncture system 1B, for example, the ultrasound probe 60 equipped with a level is brought into contact with the body surface Ma of the patient M in the vertical direction, a vertical depth D of the puncture target point Q from the body surface Ma is measured, and a target point mark Q1 is displayed at the body surface Ma immediately above the puncture target point Q. FIG. 14 shows an example in which the ultrasound probe 60 is used, and alternatively, a position of the puncture target point Q may be measured using MRI instead of the ultrasound probe 60.

Next, as shown in FIG. 15, the puncture insertion point P of the puncture needle 2 is determined based on the puncture target point Q, and an insertion point mark P1 is displayed at the body surface Ma. Thereafter, for example, the distance of the straight line L between the target point mark Q1 and the insertion point mark P1 and an angle t of the straight line L relative to the horizontal plane are measured using a length measuring instrument 61 such as a caliper. Then, the puncture angle of the puncture needle 2 is calculated based on the angle t.

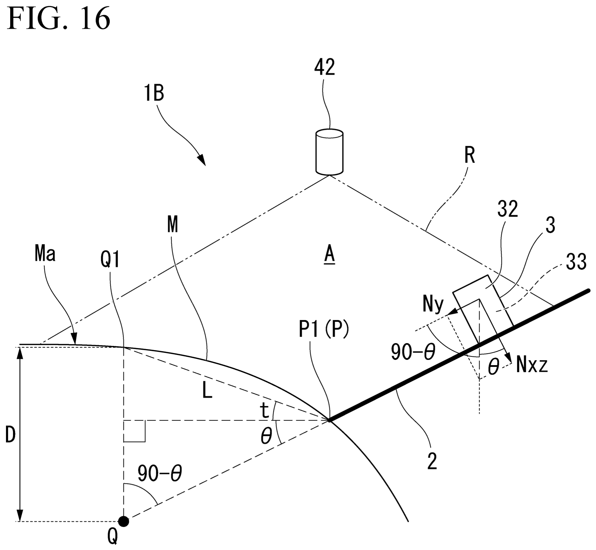

Thereafter, as shown in FIG. 16, the vertical laser plane A including the puncture insertion point P and the puncture target point Q is displayed by the laser light R emitted by the laser irradiation unit 42, and the puncture needle 2 or the sensor unit 32 of the puncture assisting tool 3 attached to the puncture needle 2 is aligned with the vertical laser plane A to match the vertical laser plane A, and thus the tilt angle of the puncture needle 2 from the vertical axis or the horizontal plane can be presented to the practitioner by the tilt angle presentation unit 33 during the puncture from when the puncture is started.

[Sixth Embodiment]

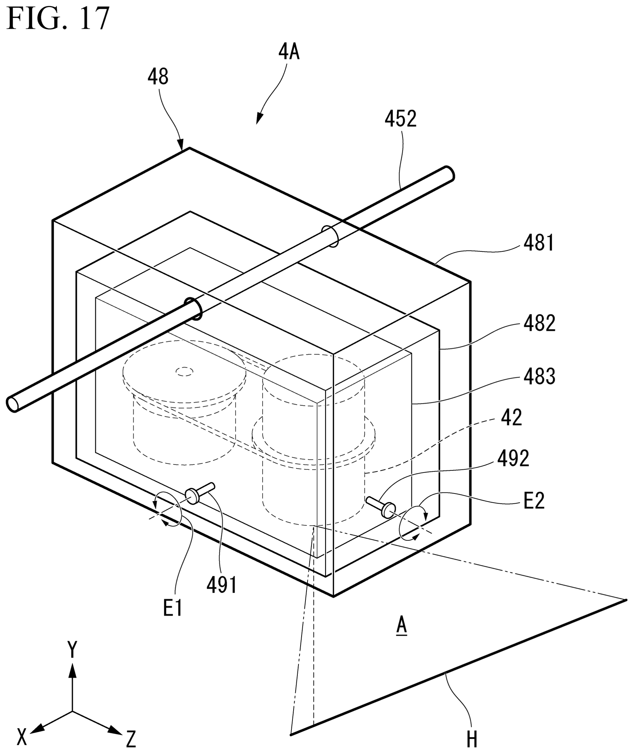

FIG. 17 shows a configuration of an adjustment mechanism 48 of a body surface-irradiating laser mechanism 4A according to a sixth embodiment. As shown in FIG. 17, the body surface-irradiating laser mechanism 4A in the sixth embodiment includes the adjustment mechanism 48 for the vertical laser plane. That is, the laser irradiation unit 42 is rotatable by the adjustment mechanism 48 about each of an X axis (first axis) along the linear direction of the horizontal frame 412 (feed screw 452) shown in FIG. 7, a Z-axis (second axis) orthogonal to the X axis, and a Y-axis (third axis) serving as an irradiation axis of the laser irradiation unit 42. The X axis is the horizontal direction as described above, and is disposed in a direction at a right angle to the body axis O of the patient M in a top view as shown in FIG. 6.

The adjustment mechanism 48 includes a first accommodation case 481 movable in the X axis, a second accommodation case 482 accommodated in the first accommodation case 481, and a third accommodation case 483 accommodated in the second accommodation case 482 to fix the laser irradiation unit 42. The first accommodation case 481, the second accommodation case 482, and the third accommodation case 483 each have a rectangular parallelepiped shape. The shape of each of the cases 481, 482, and 483 is not limited to the rectangular parallelepiped shape, and may be any shape such as a cube or a sphere.

The first accommodation case 481 is movable along the feed screw 452 extending in the X-axis direction (see FIG. 6) by an operation on the operation panel 47 or the like. The first accommodation case 481 is not rotatable around the feed screw 452.

The second accommodation case 482 is rotated around the X axis (in a direction of an arrow E1) relative to the first accommodation case 481 by a rotation drive unit such as a stepping motor due to an operation on the operation panel 47 or the like. The first accommodation case 481 and the second accommodation case 482 are supported by a first pin 491 along the X axis to be relatively rotatable around the X axis (in the E1 direction). For example, a configuration can be adopted in which a first bearing (not shown) is provided at a wall surface of the first accommodation case 481 orthogonal to the X axis, and the first pin 491 rotatably supported relative to the first bearing is fixed to the second accommodation case 482.

The third accommodation case 483 is rotated around the Z axis (in a direction of an arrow E2) relative to the second accommodation case 482 by a rotation drive unit such as a stepping motor due to an operation on the operation panel 47 or the like. The second accommodation case 482 and the third accommodation case 483 are supported by a second pin 492 along the Z axis to be relatively rotatable around the Z axis (in the E2 direction). For example, a configuration can be adopted in which a second bearing (not shown) is provided at a wall surface of the second accommodation case 482 orthogonal to the Z axis, and the second pin 492 rotatably supported relative to the second bearing is fixed to the third accommodation case 483.

The third accommodation case 483 includes an acceleration sensor (not shown). In the adjustment mechanism 48, a gravity direction can be detected based on output of the acceleration sensor provided at the third accommodation case 483, and the vertical laser plane A can be automatically calibrated vertically. The calibration is performed by automatic calibration.

Rotation directions of the second accommodation case 482 and the third accommodation case 483 may be reversed. That is, the second accommodation case 482 may rotate around the Z axis (E2 direction) relative to the first accommodation case 481, and the third accommodation case 483 may rotate around the X axis (E1 direction) relative to the second accommodation case 482.

Attachment positions of the bearings and the pins 491 and 492 can be reversed.

In this way, in the body surface-irradiating laser mechanism 4A in the sixth embodiment, even when heights of the pair of left and right leg frames 411 of the holding member 41 are different from each other as shown in FIG. 6, the horizontal frame 412 (feed screw 452) is not horizontal, and the vertical laser plane A is not vertical due to adjustment by the operation panel 47 or automatic calibration, a posture of the laser irradiation unit 42 can be calibrated such that the vertical laser plane A becomes vertical by operating the adjustment mechanism 48.

The preferred embodiments of the invention have been described above, but the invention is not limited to the embodiments, modifications, and examples. In a scope not departing from the gist of the invention, additions, omissions, replacements, and other changes to the configuration can be made.

Further, the invention is not limited to the above description, and is limited only by the appended claims.

In the embodiment, the groove portion 31 (holding portion) is provided at the movable portion 35 of the puncture assisting tool 3, and alternatively, a holding portion may be provided at the fixed portion 34, or a holding portion for fixing the puncture needle 2 may be provided at both the fixed portion 34 and the movable portion 35. The configuration of the holding portion that holds the puncture needle 2 at the sensor unit 32 is not limited to the groove portion 31, and may be another holding structure.

In the first embodiment described above, a configuration in which the body surface-irradiating laser mechanism 4 and the puncture navigation system 5 that support the laser irradiation unit 42 are provided is shown as an example, and alternatively, one or both of the body surface-irradiating laser mechanism 4 and the puncture navigation system 5 may be omitted.

Industrial Applicability

The puncture system, the puncture assisting tool, the body surface-irradiating laser mechanism, and the puncture navigation system according to the invention can be applied as a puncture system, a puncture assisting tool, a body surface-irradiating laser mechanism, and a puncture navigation system that can easily and accurately check a tilt angle of a puncture needle with which a patient is punctured and perform an accurate puncture.

Reference Signs List

    1, 1B puncture system
    2 puncture needle
    3, 3B puncture assisting tool
    4 body surface-irradiating laser mechanism
    5 puncture navigation system

31 groove portion (holding portion)
32 sensor unit
33 tilt angle presentation unit
34 fixed portion
35 movable portion
41 holding member
42 laser irradiation unit
43 movement mechanism
45 linear movement mechanism
46 rotation movement mechanism
A vertical laser plane
Ac CT cross section
Ah vertical plane
M patient
Ma body surface
P puncture insertion point
Q puncture target point
R laser light

**Claims**

1. A puncture system comprising:

a puncture assisting tool including a holding portion attached to or detached from a puncture needle that punctures using a line formed at a body surface of a patient by a vertical plane including an insertion point and a target point in a body of the patient, and a sensor unit that measures an angle of the held puncture needle from a vertical axis or a horizontal plane;
a body surface-irradiating laser mechanism including a laser irradiation unit that emits laser to the vertical plane along the line formed at the body surface of the patient, and a movement mechanism that moves the laser irradiation unit; and
a puncture navigation system including a calculation unit that determines an irradiation position of the laser and an insertion angle of the puncture needle based on the insertion point and the target point in the body of the patient, and a control unit that controls the laser irradiation unit.

2. The puncture system according to claim 1, wherein
the holding portion of the puncture assisting tool includes a fixed portion, a movable portion, and a fixing mechanism that holds and fixes the puncture needle with the fixed portion and the movable portion.

3. The puncture system according to claim 2, wherein
the fixed portion has a perpendicular surface with respect to the puncture needle when the puncture needle is fixed, and a scale is marked on the perpendicular surface.

4. The puncture system according to claim 3, wherein
the scale at the perpendicular surface has a vertical-plane-parallel scale parallel to the vertical plane and a deviation angle scale provided perpendicular to the vertical-plane-parallel scale.

5. The puncture system according to claim 1, wherein
the movement mechanism includes a rotation movement mechanism that rotatably holds the laser irradiation unit.

6. The puncture system according to claim 1, wherein
the movement mechanism includes a linear movement mechanism that holds the laser irradiation unit movably in a linear direction.

7. The puncture system according to claim 6, wherein
the laser irradiation unit is rotatably provided around each of a first axis along the linear direction, a second axis orthogonal to the first axis, and a third axis forming an irradiation axis of the laser irradiation unit.

8. The puncture system according to claim 1, wherein
the calculation unit determines an insertion angle and an insertion length based on the target point and the insertion point, and calculates an angle from a body axis of the patient relative to the vertical plane.

9. The puncture system according to claim 1, further comprising:
at least one of CT, MRI, and an ultrasound probe that identify the target point in the body of the patient.

10. A puncture assisting tool attachable to and detachable from a puncture needle that punctures using a line formed at a body surface of a patient by a vertical plane including an insertion point and a target point in a body of the patient, the puncture assisting tool comprising:

a holding portion including a fixed portion that holds the puncture needle, a movable portion, and a fixing mechanism that holds and fixes the puncture needle with the fixed portion and the movable portion;
a sensor unit configured to measure an angle of the held puncture needle from a vertical axis or a horizontal plane; and
a presentation unit configured to present the angle, wherein
the fixed portion has a perpendicular surface with respect to the puncture needle when the puncture needle is fixed, and a scale is marked on the perpendicular surface.

11. A body surface-irradiating laser mechanism comprising:

a holding member;
a laser irradiation unit attached via the holding member and configured to emit laser along a line formed at a body surface of a patient by a vertical plane including an insertion point and a target point in a body of the patient toward the vertical plane; and
a movement mechanism configured to move the laser irradiation unit provided at the holding member.

12. A puncture navigation system comprising:

a calculation unit configured to determine an insertion angle and an insertion length based on an insertion point and a target point in a body of a patient specified using at least one of CT, MRI, and an ultrasound probe, and to calculate an angle from a body axis of the patient relative to a vertical plane including the target point and the insertion point; and
a control unit configured to control, based on the angle from the body axis of the patient calculated by the calculation unit, a laser irradiation unit that emits laser to the vertical plane along a line formed at a body surface of the patient by the vertical plane.

FIG. 1

EP 4 656 154 A1

FIG. 2

FIG. 3

FIG. 4

FIG. 5

FIG. 6

EP 4 656 154 A1

FIG. 7

# FIG. 8

FIG. 9

FIG. 10

FIG. 11

FIG. 12

FIG. 13

FIG. 14

FIG. 15

FIG. 16

## FIG. 17

## INTERNATIONAL SEARCH REPORT

| | International application No. |
|---|---|
| | **PCT/JP2023/047014** |

**A. CLASSIFICATION OF SUBJECT MATTER**

***A61B 34/20***(2016.01)i
FI: A61B34/20

According to International Patent Classification (IPC) or to both national classification and IPC

**B. FIELDS SEARCHED**

Minimum documentation searched (classification system followed by classification symbols)

A61B34/20

Documentation searched other than minimum documentation to the extent that such documents are included in the fields searched

Published examined utility model applications of Japan 1922-1996
Published unexamined utility model applications of Japan 1971-2024
Registered utility model specifications of Japan 1996-2024
Published registered utility model applications of Japan 1994-2024

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
|---|---|---|
| Y | US 2016/0296179 A1 (PRECISIONRAD LLC) 13 October 2016 (2016-10-13) paragraphs [0027]-[0040], fig. 1-3C | 1-2, 5-9 |
| A | | 3-4, 10 |
| X | US 5782842 A (DAUM GMBH) 21 July 1998 (1998-07-21) column 3, lines 47-54, column 4, lines 30-48, column 4, line 64 to column 5, line 14, fig. 1, 3 | 11-12 |
| Y | | 1-2, 5-9 |
| A | | 3-4, 10 |
| A | WO 2020/182279 A1 (SIEMENS HEALTHCARE GMBH) 17 September 2020 (2020-09-17) entire text, all drawings | 1-12 |
| A | JP 10-337290 A (SIEMENS AKTIENGESELLSCHAFT) 22 December 1998 (1998-12-22) entire text, all drawings | 1-12 |

☐ Further documents are listed in the continuation of Box C.    ☑ See patent family annex.

| | | | |
|---|---|---|---|
| * | Special categories of cited documents: | "T" | later document published after the international filing date or priority date and not in conflict with the application but cited to understand the principle or theory underlying the invention |
| "A" | document defining the general state of the art which is not considered to be of particular relevance | | |
| "D" | document cited by the applicant in the international application | "X" | document of particular relevance; the claimed invention cannot be considered novel or cannot be considered to involve an inventive step when the document is taken alone |
| "E" | earlier application or patent but published on or after the international filing date | | |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | "&" | document member of the same patent family |
| "P" | document published prior to the international filing date but later than the priority date claimed | | |

| Date of the actual completion of the international search | Date of mailing of the international search report |
|---|---|
| **22 February 2024** | **19 March 2024** |

| Name and mailing address of the ISA/JP | Authorized officer |
|---|---|
| **Japan Patent Office (ISA/JP)** <br> **3-4-3 Kasumigaseki, Chiyoda-ku, Tokyo 100-8915** <br> **Japan** | |
| | Telephone No. |

Form PCT/ISA/210 (second sheet) (July 2022)

**INTERNATIONAL SEARCH REPORT**

International application No.

**PCT/JP2023/047014**

| **Box No. III** | **Observations where unity of invention is lacking (Continuation of item 3 of first sheet)** |
|---|---|

This International Searching Authority found multiple inventions in this international application, as follows:

Document 1: US 2016/0296179 A1 paragraphs [0027]-[0040], fig. 1-3C

Claims are classified into four inventions below.

(Invention 1) Claims 1-9
Claims 1-9 have the special technical feature of a "puncture system comprising: a puncture assisting tool including a holding unit that is detachably attached to a puncture needle with which puncturing is performed by using a line formed by a surface including a target point inside a body of a patient and an insertion point, the line being formed on a body surface of the patient, and a sensor unit that is able to measure an angle from a vertical axis or a horizontal surface of the held puncture needle; a body surface irradiation laser mechanism including a laser irradiation unit that emits a laser beam along the line formed by the surface on the body surface of the patient and a moving mechanism that moves the laser irradiation unit; and a puncture navigation system including a calculation unit that determines an irradiation position with the laser beam and an insertion angle of the puncture needle from the target point inside the body of the patient and the insertion point, and a control unit that controls the laser irradiation unit," and are thus classified as invention 1.

(Invention 2) Claim 10
Claim 10 shares, with claim 1 classified as invention 1, the common technical feature of a "puncture assisting tool including a holding unit that is detachably attached to a puncture needle which performs puncturing by using a line formed by a surface including a target point inside a body of a patient and an insertion point, the line being formed on a body surface of the patient, and a sensor unit that is able to measure an angle from a vertical axis or a horizontal surface of the held puncture needle." However, said technical feature does not make a contribution over the prior art in light of the disclosure of document 1 (in particular, see paragraphs [0027]-[0028], fig. 1-2B), and thus cannot be said to be a special technical feature. There are no other same or corresponding special technical features between claim 10 and claim 1.
Claim 10 is not dependent on claim 1. Claim 9 is not substantially identical to or similarly closely related to any of the claims classified as invention 1.
Therefore, claim 10 cannot be classified as invention 1.
Claim 10 has the special technical feature of a "puncture assisting tool that is detachably attached to a puncture needle which performs puncturing by using a line formed by a surface including a target point inside a body of a patient and an insertion point, the line being formed on a body surface of the patient, the puncture assisting tool comprising: a holding unit including a fixing portion that holds the puncture needle, a movement portion, and a fixing mechanism that holds the puncture needle by using the fixing portion and the movement portion to fix the puncture needle; a sensor unit that is able to measure an angle from a vertical axis or a horizontal surface of the held puncture needle; and a presentation unit that present the angle, wherein the fixing portion has a perpendicular surface perpendicular to the puncture needle and the perpendicular surface has a scale when the puncture needle is fixed," and is thus classified as invention 2.

(Invention 3) Claim 11
It cannot be said that claim 11 has the special technical feature identical or corresponding to claim 1 classified as invention 1 or claim 10 classified as invention 2.
Claim 11 is not dependent on any of claims 1 and 10. Claim 11 is not substantially identical to or similarly closely related to any of the claims classified as invention 1, or invention 2.
Therefore, claim 11 cannot be classified as either invention 1 or invention 2.
Claim 11 does not have a special technical feature, and is thus classified as invention 3.

(Invention 4) Claim 12
It cannot be said that claim 12 has the special technical feature identical or corresponding to claim 1 classified as invention 1, claim 10 classified as invention 2, and claim 11 classified as invention 3.
Claim 12 is not dependent on any of claims 1, 10, and 11. Claim 12 is not substantially identical to or similarly closely related to any of the claims classified as invention 1, invention 2, or invention 3.
Therefore, claim 12 cannot be classified as any of inventions 1, 2, and 3.
Claim 12 does not have a special technical feature, and is thus classified as invention 4.

Form PCT/ISA/210 (continuation of first sheet) (July 2022)

## INTERNATIONAL SEARCH REPORT

| International application No. |
|---|
| **PCT/JP2023/047014** |

**Box No. III      Observations where unity of invention is lacking (Continuation of item 3 of first sheet)**

1. ☐ As all required additional search fees were timely paid by the applicant, this international search report covers all searchable claims.

2. ☑ As all searchable claims could be searched without effort justifying additional fees, this Authority did not invite payment of additional fees.

3. ☐ As only some of the required additional search fees were timely paid by the applicant, this international search report covers only those claims for which fees were paid, specifically claims Nos.:

4. ☐ No required additional search fees were timely paid by the applicant. Consequently, this international search report is restricted to the invention first mentioned in the claims; it is covered by claims Nos.:

**Remark on Protest**      ☐ The additional search fees were accompanied by the applicant's protest and, where applicable, the payment of a protest fee.

☐ The additional search fees were accompanied by the applicant's protest but the applicable protest fee was not paid within the time limit specified in the invitation.

☐ No protest accompanied the payment of additional search fees.

Form PCT/ISA/210 (continuation of first sheet) (July 2022)

## INTERNATIONAL SEARCH REPORT
### Information on patent family members

International application No.

**PCT/JP2023/047014**

| Patent document cited in search report | | | Publication date (day/month/year) | Patent family member(s) | | | Publication date (day/month/year) |
|---|---|---|---|---|---|---|---|
| US | 2016/0296179 | A1 | 13 October 2016 | (Family: none) | | | |
| US | 5782842 | A | 21 July 1998 | DE | 19501069 | A1 | |
| WO | 2020/182279 | A1 | 17 September 2020 | (Family: none) | | | |
| JP | 10-337290 | A | 22 December 1998 | US | 6041249 | A | |
| | | | | entire text, all drawings | | | |
| | | | | DE | 19801446 | A1 | |
| | | | | CN | 1198918 | A | |

Form PCT/ISA/210 (patent family annex) (July 2022)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2023002678 W
- JP 2002511784 A
- JP 2000070272 A
- JP 2009523508 A
- CN 1939234 B

- WO 2017070124 A
- US 20100030082 A
- US 20160296179 A
- US 5782842 B